# EUROPEAN PATENT SPECIFICATION

(11) **EP 2 457 902 B1**
(45) Date of publication and mention of the grant of the patent: **02.09.2015**
(21) Application number: 10802514.9
(22) Date of filing: 12.07.2010
(51) Int. Cl.: C07D 251/60, C07D 251/62

(54) **METHOD FOR PRODUCING AND PURIFYING MELAMINE**
VERFAHREN ZUR HERSTELLUNG UND REINIGUNG VON MELAMIN
PROCÉDÉ DE PRODUCTION ET DE PURIFICATION DE MÉLAMINE

(30) Priority: 20.07.2009 RU 2009127905
(43) Date of publication of application: 30.05.2012
(73) Proprietor: Otkrytoe Aktsionernoe Obschestvo Research & Design Institute Of Urea And Organic Synthesis Products (OAO NIIK), Nizhny Novgorod Region 606008 (RU)
(72) Inventor: NIKOLAEV, Evgeny Yuryevich, Dzerzhinsk Nizhny Novgorodskaya obl. 606037 (RU); SKUDIN, Aleksei Georgievich, Dzerzhinsk Nizhny Novgorodskaya obl. 606009 (RU); SOLDATOV, Aleksei Vladimirovich, Dzerzhinsk Nizhny Novgorodskaya obl. 606025 (RU); PROKOPYEV, Aleksandr Alekseevich, Dzerzhinsk Nizhny Novgorodskaya obl. 606008 (RU); KUZNETSOV, Nikolai Mikhailovich, Dzerzhinsk Nizhny Novgorodskaya obl. 606000 (RU); KOSTIN, Oleg Nikolaevich, Nizhny Novgorod 603010 (RU); ESIN, Igor Veniaminovich, Dzerzhinsk Nizhny Novgorodskaya obl. 606000 (RU)
(74) Representative: Vossius & Partner Patentanwälte Rechtsanwälte mbB
(86) International application number: PCT/RU2010/000386
(87) International publication number: WO 2011/010953

(56) References cited:
- WO-A1-01/36397
- WO-A1-96/20183
- WO-A1-97/20826
- WO-A1-03/045927
- GB-A- 958 633
- GB-A- 958 633
- RU-C2- 2 161 610
- RU-C2- 2 271 354

## Description

### Field of the Invention

The invention relates to the technology of melamine production by converting urea into melamine using a non-catalytic method at high pressure, as a whole, as well as to one of melamine recovery stages using aqueous solutions, especially to the stage of dissolved gases removal from an aqueous solution of crude melamine, and can be used for commercial melamine production.

### Background of the Invention

The methods of melamine production are known which include a synthesis stage by means of conversion of urea into melamine at increased temperature and pressure and melamine recovery stages using aqueous solutions, including a stage of dissolved gases removal from an aqueous solution of crude melamine (Maxwell G., Synthetic Nitrogen Products. A Practical Guide to the Products and Processes. Springer, 2004, pp. 300-302; Nitrogen, 1982, No. 139, pp. 32-39).

Using these methods, a reaction mixture is formed at synthesis stage that contains, apart from the main substance, a significant amount of impurities consisting mainly of melamine condensation products and oxyaminotriazines. A substantial part of the produced melamine is used to make melamine-formaldehyde resins applied for lacquers production including furniture lacquers. One of the main properties of these lacquers is their transparency. To produce a high quality lacquer, the melamine quality should meet strict requirements: 99.8% min. of main substance content and ability to form transparent resins. The above mentioned impurities have negative influence on the resins transparency, that is why the development of melamine recovery stages providing high level of product purity is an up-to-date task.

From the technical point of view, the method that is the closest to the proposed method of melamine production is the known one comprising the conversion of urea into melamine at increased temperature and pressure with the formation of a gas-liquid reaction mixture containing melamine, ammonia, carbon dioxide and impurities, cooling of gas-liquid reaction mixture at lowering pressure and contact with an aqueous solution containing ammonia and carbon dioxide (ammonium carbonate solution), with the formation of an aqueous solution of crude melamine and the partial removal of dissolved gases, allowing of obtained melamine solution to stand in order to decompose a part of impurities, subsequent lowering pressure and temperature and removal of dissolved gases from an aqueous solution of crude melamine by means of stripping with water vapour, addition of sodium hydroxide to the melamine solution produced and separating melamine from the solution by crystallization (GB 958633, C07d, 1964, Example 3).

The above patent discloses several methods of melamine production that differ in stages of main product separation. The prototype is Example 3 including the stage of dissolved gases removal from an aqueous solution of crude melamine by its stripping with water vapour. The finished product obtained by this method contains only 99.0% of melamine and a considerable amount of impurities: 0.7% of oxyaminotriazines and 0.2% of condensation products. The test results melamine having such quality show that melamine-formaldehyde resin produced from this melamine is not transparent and cannot be used for lacquering.

Moreover, the experience of commercial operation of melamine production based on the above method showed an occasional sharp degradation of melamine purity in case of fluctuations of process parameters before the stripping with water vapour. It is caused by increase of impurities content in the product (oxyaminotriazines and condensation products), and this disadvantage cannot be eliminated with the help of the melamine recovery stages used in this method.

Therefore, the described method of melamine production cannot provide a target product of high transparency independent of process parameters fluctuations.

From the technical point of view the method that is the closest to the proposed method of removing dissolved gases from an aqueous solution of crude melamine is a known method of the solution stripping with water vapour (GB 958633, C07d, 1964, example 3).

Usage of this method in melamine production technology does not allow to get a target product of high purity and to provide product purity with its independence from process parameters fluctuations.

### Summary of the Invention

Improvement of melamine production method ensuring the production of a high quality target product is the technical task for this invention to solve.

A technical result that could be obtained by means of the invention is melamine purity enhancement and its independence from the process parameters fluctuations.

To achieve the technical result, the melamine production method is proposed which includes conversion of urea into melamine at increased temperature and pressure with the formation of a gas-liquid reaction mixture containing melamine, ammonia, carbon dioxide and impurities, cooling of the gas-liquid reaction mixture while lowering pressure and contact with an aqueous medium resulting in the formation of an aqueous solution of crude melamine and the partial removal of dissolved gases, allowing the produced melamine solution to stand in order to decompose a part of impurities, subsequently lowering the temperature and the pressure and the removal of dissolved gases from the aqueous solution of crude melamine by means of stripping with water vapour, the addition of an alkali metal hydroxide solution to the obtained melamine solution, the separation of melamine from the solution by crystallization, characterized in that ammonia is introduced additionally in the process of stripping with water vapour, wherein the ammonia is introduced in amount of 0.01-1.0 kg per 1 kg of melamine, and wherein the stripping with water vapour is carried out at 100-150°C and under a pressure of 0.1-0.6 MPa.

Due to ammonia introduction into water vapour stripping process of the crude melamine aqueous solution, the proposed method of melamine production assures stable high quality of the target product with the purity of 99.8% regardless of process parameters fluctuations prior to water vapour stripping. Melamine-formaldehyde resins based on melamine produced by means of the proposed method, have high transparency. The proposed procedure is not described in the prior art and is novel.

### Description of the Preferred Embodiments

The concept of invention is illustrated below by the specific examples of realization of the proposed melamine production method using the proposed method for removal of dissolved gases from the aqueous solution of crude melamine.

### Example 1.

Melamine synthesis is carried out by conversion of urea into melamine in a reactor-autoclave at 375°C and the pressure of 7 MPa. Reaction mass containing 23% of melamine, 45% of ammonia, 28% of carbon dioxide and 4% of impurities, is cooled in a quenching device down to 160°C with ammonium carbonate solution containing 17% of ammonia and 4% of carbon dioxide (9 kg of ammonium carbonate solution per 1 kg of melamine), lowering pressure simultaneously down to 2.5 MPa. Reaction gases are discharged from the upper part of the quenching device. Formed melamine solution is sent to the column type vessel where it is allowed to stand for 70 minutes in order to decompose a part of impurities, and then it is transferred to the plate-type stripper with pressure lowering down to 0.1 MPa and the temperature down to 100°C. Steam and gaseous ammonia are supplied to the stripper bottom and to the part of the lower trays in amount of 3 kg and 0.1 kg per 1 kg of melamine, correspondingly. The trays are supplied with the mother liquor formed as a result of centrifugal separation of melamine crystals. Sodium hydroxide solution is introduced into the stripper bottom in a quantity sufficient to obtain sodium hydroxide concentration of 0.5%. The solution at the outlet from the stripper has about 4% of melamine, 0.2% of ammonia and less than 0.01% of carbon dioxide. The solution is purified at 100°C in a cardboard and carbon filters, and melamine is recovered by means of crystallization at 40°C. After that, melamine crystals are separated by centrifugal process, washed with water and dried.

Finished product contains 99.9% of the main substance, 0.01% of condensation products and less than 0.001 % of oxyaminotriazines. Melamine-formaldehyde resin produced from this melamine sample is transparent.

### Example 2.

Melamine synthesis is carried out as in the Example 1 but it takes place under the following conditions. The conversion of urea into melamine occurs at 400°C and the pressure of 10 MPa. Reaction mass containing 27.5% of melamine, 38% of ammonia, 30% of carbon dioxide and 4.5% of impurities, is cooled in the quenching device down to 170°C with ammonium carbonate solution containing 17% of ammonia and 4% of carbon dioxide (10 kg of ammonium carbonate solution per 1 kg of melamine) lowering pressure simultaneously down to 3 MPa. Reaction gases are removed from the upper part of the quenching device. Formed melamine solution is transferred to the column type vessel where it is allowed to stand for 40 minutes, and then it is transferred to the plate-type stripper with lowering pressure down to 0.3 MPa and the temperature down to 120°C. Steam in amount of 2.5 kg per 1 kg of melamine is supplied to the stripper bottom and to the part of lower trays, and the central trays are supplied with liquid ammonia in amount of 0.01 kg per 1 kg of melamine. The trays are supplied with the mother liquor formed as a result of centrifugal separation of melamine crystals. Sodium hydroxide solution is introduced into the bottom of the stripper in the amount sufficient to obtain sodium hydroxide concentration of 0.5%. The solution at the outlet from the stripper has about 5% of melamine, less than 0.01% of ammonia and 0.1% of carbon dioxide. Melamine is separated from the solution by means of crystallization at 38°C. After that, melamine crystals are separated by centrifugal process, washed with water and dried.

Finished product contains 99.9% of the main substance, 0.01% of condensation products and 0.01% of oxyaminotriazines. Melamine-formaldehyde resin produced from this melamine sample is transparent.

### Example 3.

Melamine synthesis is carried out as in the Example 1 but it takes place under the following conditions. The conversion of urea into melamine occurs at 390°C and the pressure of 10 MPa. Reaction mass containing 28% of melamine, 38% of ammonia, 30% of carbon dioxide and 4% of impurities, is cooled in the quenching device down to 160°C with ammonium carbonate solution containing 17% of ammonia and 4% of carbon dioxide (11 kg of ammonium carbonate solution per 1 kg of melamine) lowering pressure simultaneously down to 2.4 MPa. Reaction gases are removed from the upper part of the quenching device. Formed melamine solution is transferred to the column type vessel where it is allowed to stand for 60 minutes and then it is transferred to the plate-type stripper with lowering pressure down to 0.6 MPa and the temperature down to 150°C. Steam in amount of 3 kg per 1 kg of melamine is supplied to the stripper bottom whereas 25% aqueous ammonia solution in amount of 2.0 kg per 1 kg of melamine is supplied to the central part of the stripper. Sodium hydroxide solution in the amount sufficient to obtain sodium hydroxide concentration of 0.2% is introduced into the bottom of the stripper. The solution at the outlet from the stripper has about 6% of melamine, less than 0.01% of ammonia and less than 0.01% of carbon dioxide. The solution is purified at 120°C in cardboard and carbon filters, and then melamine is separated by means of crystallization at 40°C. After that, melamine crystals are separated by centrifugal process, washed with water and dried.

Finished product contains 99.9% of the main substance, less than 0.001% of condensation products and 0.02% of oxyaminotriazines. Melamine-formaldehyde resin produced on the basis of this melamine sample is transparent.

### Example 4.

Melamine synthesis is carried out as in the Example 1 but it takes place under the following conditions. The conversion of urea into melamine occurs at 385°C and the pressure of 8 MPa. Reaction mass containing 27% of melamine, 35% of ammonia, 33% of carbon dioxide and 5% of impurities, is cooled in the quenching device down to 160°C with ammonium carbonate solution containing 17% of ammonia and 4% of carbon dioxide (10 kg of ammonium carbonate solution per 1 kg of melamine) lowering pressure simultaneously down to 2.5 MPa. Reaction gases are removed from the upper part of the quenching device. Melamine solution produced is transferred to the column type vessel where it is allowed to stand for 70 minutes, and then it is transferred to the plate-type stripper with lowering pressure down to 0.1 MPa and the temperature down to 100°C. Steam and gaseous ammonia are supplied to the stripper bottom and to the part of lower trays, in amount of 2.8 kg and 0.5 kg per 1 kg of melamine, correspondingly. Additionally, 25% aqueous ammonia solution in amount of 0.4 kg per 1 kg of melamine is supplied to the upper tray. The trays are supplied with the mother liquor formed as a result of centrifugal separation of melamine crystals. Sodium hydroxide solution in the amount sufficient to obtain sodium hydroxide concentration of 0.2% is introduced into the bottom of the stripper. The solution at the outlet from the stripper has about 4% of melamine, 0.1% of ammonia and 0.2% of carbon dioxide. The solution is purified at 100°C in cardboard and carbon filters, and then melamine is separated by means of crystallization at 38°C. After that, melamine crystals are separated by centrifugal process, washed with water and dried.

Finished product contains 99.9% of the main substance, 0.01% of condensation compounds and 0.01% of oxyaminotriazines. Melamine-formaldehyde resin produced from this melamine sample is transparent.

### Example 5

Melamine synthesis is carried out as in the Example 1 but it takes place under the following conditions. The conversion of urea into melamine occurs at 365°C and the pressure of 7 MPa. Reaction mass containing 27% of melamine, 38% of ammonia, 30% of carbon dioxide and 5% of impurities, is cooled in the quenching device down to 150°C with ammonium carbonate solution containing 17% of ammonia and 4% of carbon dioxide (9 kg of ammonium carbonate solution per 1 kg of melamine) lowering simultaneously pressure down to 2.2 MPa. Reaction gases are removed from the upper part of the quenching device. Produced melamine solution is transferred to the column type vessel where it is allowed to stand for 65 minutes, and then it is transferred to the plate-type stripper with lowering pressure down to 0.3 MPa and the temperature down to 120°C. Steam in amount of 2 kg per 1 kg of melamine is supplied to the stripper bottom and to the part of lower trays, liquid ammonia in amount of 0.01 kg per 1 kg of melamine is supplied to the central trays, and gaseous ammonia in amount of 0.6 kg per 1 kg of melamine is supplied to the lower trays. The trays are supplied by steam condensate (water). Sodium hydroxide solution in the amount sufficient to obtain sodium hydroxide concentration of 0.05% is introduced into the bottom of the stripper. The solution at the outlet from the stripper has about 5% of melamine, 0.1% of ammonia and 0.1% of carbon dioxide. The solution is purified at 100°C in cardboard and carbon filters, and then melamine is separated by means of crystallization at 38°C. After that, melamine crystals are separated by centrifugal process, washed with water and dried.

Finished product contains 99.9% of the main substance, less than 0.001% of condensation products and 0.01% of oxyaminotriazines. Melamine-formaldehyde resin produced from this melamine sample is transparent.

### Example 6.

Melamine synthesis is carried out as in the Example 1 but it takes place under the following conditions. The conversion of urea into melamine occurs at 380°C and the pressure of 9 MPa. Reaction mass containing 28% of melamine, 37.5% of ammonia, 30% of carbon dioxide and 4.5% of impurities, is cooled in the quenching device down to 165°C with water (10 kg of water per 1 kg of melamine) lowering simultaneously pressure down to 2.8 MPa. Reaction gases are removed from the upper part of the quenching device. Formed melamine solution is transferred to the column type vessel where it is allowed to stand for 50 minutes, and then it is transferred to the plate-type stripper with lowering pressure down to 0.3 MPa and the temperature down to 120°C. Steam in amount of 3 kg per 1 kg of melamine is supplied to the stripper bottom and to the part of lower trays, and gaseous ammonia in amount of 1 kg per 1 kg of melamine is supplied to the lower trays. The plates are supplied with the mother liquor obtained as a result of centrifugal separation of melamine crystals. Potassium hydroxide solution in the amount sufficient to obtain potassium hydroxide concentration of 0.1 % is introduced into the bottom of the stripper. The solution at the outlet from the stripper has about 5% of melamine, less than 0.01% of ammonia and 0.2% of carbon dioxide. The solution is purified at 110°C in cardboard and carbon filters, and then melamine is separated by means of crystallization at 40°C. After that, melamine crystals are separated by centrifugal process, washed with water and dried.

Finished product contains 99.9% of the main substance, 0.005% of condensation products and 0.01% of oxyaminotriazines. Melamine-formaldehyde resin produced from this melamine sample is transparent.

Example 7 (according to the prototype). Melamine synthesis by conversion of urea into melamine is carried out in the reactor at 380°C and the pressure of 10 MPa. Reaction mass containing 26% of melamine (including impurities), 46% of ammonia and 28% of carbon dioxide is cooled in the quenching device down to 150°C with an aqueous solution containing 25% of ammonia and 8% of carbon dioxide (ammonium carbonate solution) in amount of 9 kg of solution per 1 kg of melamine lowering pressure simultaneously down to 3 MPa. Reaction gases are removed from the upper part of the quenching device. Melamine solution is allowed to stand for 60 minutes in the quenching device, and then it is transferred to the plate-type stripper with lowering pressure down to 0.1 MPa and the temperature down to 100°C. Steam in amount of 0.25 kg per 1 kg of melamine solution entering the stripper is supplied to the stripper bottom. Melamine suspension is transferred to another vessel where melamine is dissolved in the mother liquor containing 0.2% of sodium hydroxide. Melamine concentration in the produced solution is approximately 5% at 100°C. The solution is filtered at 100°C and transferred to the crystallizer where it is cooled down to 25°C. Formed melamine crystals are separated by centrifugal process, washed with one-and-a-half amount of water and dried. Produced melamine contains 99.0% of the main substance, 0.7% of oxyaminotriazines and 0.2% of condensation compounds. Melamine-formaldehyde resin produced from this melamine sample is not transparent.

### Industrial Applicability

The invention can be used for commercial production of melamine.

**Table**

| Parameter | Synthesis conditions of the Examples | | | | | | |
|---|---|---|---|---|---|---|---|
| | 1 | 2 | 3 | 4 | 5 | 6 | 7 (according to the prototype) |
| **Conversion of urea into melamine** | | | | | | | |
| temperature, °C | 375 | 400 | 390 | 385 | 365 | 380 | 380 |
| pressure, MPa | 7 | 10 | 10 | 8 | 7 | 9 | 10 |
| **Reaction mixture composition**, % | | | | | | | |
| melamine | 23 | 27.5 | 28 | 27 | 27 | 28 | 26 |
| ammonia | 45 | 38 | 38 | 35 | 38 | 37.5 | 46 |
| carbon dioxide | 28 | 30 | 30 | 33 | 30 | 30 | 28 |
| impurities | 4 | 4.5 | 4 | 5 | 5 | 4.5 | As part of melamine |
| **Mixture cooling** | | | | | | | |
| temperature, °C | 160 | 170 | 160 | 160 | 150 | 165 | 150 |
| pressure, MPa | 2.5 | 3.0 | 2.4 | 2.5 | 2.2 | 2.8 | 3.0 |
| **Mixture allowing to stand** | | | | | | | |
| temperature, °C | 160 | 170 | 160 | 160 | 150 | 165 | 150 |
| pressure, MPa | 2.5 | 3.0 | 2.4 | 2.5 | 2.2 | 2.8 | 3.0 |
| time, minutes | 70 | 40 | 60 | 70 | 65 | 50 | 60 |
| **Separation of dissolved gases** | | | | | | | |
| temperature, °C | 100 | 120 | 150 | 100 | 120 | 120 | 100 |
| pressure, MPa | 0.1 | 0.3 | 0.6 | 0.1 | 0.3 | 0.3 | 0.1 |
| Amount of introduced ammonia, kg/ 1 kg of melamine | 0.1 | 0.01 | 0.5 | 0.6 | 0.61 | 1 | - |
| **Content in produced solution, %** | | | | | | | |
| ammonia | 0.2 | < 0.01 | < 0.01 | 0.1 | 0.1 | < 0.01 | - |
| carbon dioxide | < 0.01 | 0.1 | < 0.01 | 0.2 | 0.1 | 0.2 | - |
| **Product composition, %** | | | | | | | |
| melamine | 99.9 | 99.9 | 99.9 | 99.9 | 99.9 | 99.9 | 99.0 |
| condensation products | 0.01 | 0.01 | < 0.001 | 0.01 | < 0.001 | 0.005 | 0.2 |
| oxyaminotriazines | < 0.001 | 0.01 | 0.02 | 0.01 | 0.01 | 0.01 | 0.7 |
| **Melamine based resin** | | | | | | | |
| Transparency | yes | yes | yes | yes | yes | yes | no |

## Claims

1. A method for producing melamine including conversion of urea into melamine at increased temperature and pressure with the formation of a gas-liquid reaction mixture containing melamine, ammonia, carbon dioxide and impurities, cooling of the gas-liquid reaction mixture while lowering pressure and contact with an aqueous medium resulting in the formation of an aqueous solution of crude melamine and the partial removal of dissolved gases, allowing the produced melamine solution to stand in order to decompose a part of impurities, subsequently lowering the temperature and the pressure and the removal of dissolved gases from the aqueous solution of crude melamine by means of stripping with water vapour, the addition of an alkali metal hydroxide solution to the obtained melamine solution, the separation of melamine from the solution by crystallization, **characterized in that** ammonia is introduced additionally in the process of stripping with water vapour,
wherein the ammonia is introduced in amount of 0.01-1.0 kg per 1 kg of melamine, and wherein the stripping with water vapour is carried out at 100-150°C and under a pressure of 0.1-0.6 MPa.

2. The method according to claim 1 **characterized in that** ammonia is introduced in a gaseous and/or liquid state and/or in the form of aqueous solution.

3. The method for removal of dissolved gases from an aqueous solution of crude melamine by its stripping with water vapour **characterized in that** ammonia is introduced additionally in the process of stripping with water vapour,
wherein the ammonia is introduced in amount of 0.01-1.0 kg per 1 kg of melamine, and
wherein the stripping with water vapour is carried out at 100-150°C and the pressure of 0,1-0,6 MPa.

4. The method according to claim 3 **characterized in that** ammonia is introduced in a gaseous and/or liquid state and/or in the form of an aqueous solution.

## Patentansprüche

1. Verfahren zur Melaminherstellung, umfassend Umwandlung von Harnstoff in Melamin bei erhöhten Temperaturen und Druck mit Bildung eines Gas-Flüssigkeit-Reaktionsgemisches, das Melamin, Ammoniak, Kohlendioxid und Verunreinigungen enthält; Kühlung des Gas-Flüssigkeit-Reaktionsgemisches bei Druckerniedrigung und im Kontakt mit wässrigem Medium, wobei eine wässrige Lösung von Rohmelamin gebildet wird und ein Teil der gelösten Gase entfernt wird; Stehenlassen der hergestellten Melamin-Lösung für das Zersetzen eines Teiles der Verunreinigungen; nachfolgende Druck- und Temperaturerniedrigung und Entfernung der gelösten Gase aus der wässrigen Lösung des Rohmelamins durch Strippen mit Wasserdampf; das Zugeben einer Alkalimetallhydroxidlösung zur erhaltenen Melaminlösung, Abtrennung des Melamins aus der Lösung durch Kristallisierung, **dadurch gekennzeichnet, dass** beim Strippen mit Wasserdampf zusätzlich Ammoniak eingebracht wird, wobei der Ammoniak in einer Menge von 0,01-1,0 kg pro 1 kg des Melamins eingebracht wird und das Strippen mit Wasserdampf bei 100-150°C und unter einem Druck von 0,1-0,6 MPa durchgeführt wird.

2. Verfahren nach Anspruch 1, **dadurch gekennzeichnet, dass** Ammoniak in Gas- und/oder flüssiger Form und/oder in Form einer wässrigen Lösung eingebracht wird.

3. Verfahren zur Entfernung von gelösten Gasen aus einer wässrigen Lösung von Rohmelamin durch Strippen mit Wasserdampf, **dadurch gekennzeichnet, dass** beim Strippen mit Wasserdampf zusätzlich Ammoniak eingebracht wird, wobei der Ammoniak in einer Menge von 0,01-1,0 kg pro 1 kg des Melamins eingebracht wird und das Strippen mit Wasserdampf bei 100-150°C und unter einem Druck von 0,1-0,6 MPa durchgeführt wird.

4. Verfahren nach Anspruch 3, **dadurch gekennzeichnet, dass** Ammoniak in Gas- und/oder flüssiger Form und/oder in Form von einer wässrigen Lösung eingebracht wird.

## Revendications

1. Une méthode de la production de mélamine comprenant la transformation de l'urée en mélamine sous température et pression élevée avec la formation d'un mélange réactionnel de gaz et de liquide contenant de la mélamine, de la ammoniaque, du dioxide de carbone et des impuretés, le refroidissement du mélange réactionnel de gaz et de liquide en abaissant la pression et en contact avec le milieu aqueux avec la formation d'une solution aqueuse de mélamine non purifiée et l'extraction d'une partie de gaz dissous, le maintien de la solution de mélamine obtenue pour la décomposition d'une partie d'impuretés, l'abaissement ultérieur de la pression et de la température et l'extraction des gaz dissous à partir de la solution aqueuse de mélamine non purifiée par soufflage avec de la vapeur d'eau, l'addition d'une solution d'hydroxyde de métal alcalin à la solution de mélamine obtenue et la séparation de la mélamine à partir de la solution par cristallisation, **caractérisé par le fait qu'**au cours du soufflage par la vapeur d'eau on ajoute en plus de l'ammoniac où l'ammoniac est ajouté en quantité de 0,01-1,0 kg / 1 kg de mélamine et où le soufflage par la vapeur d'eau est effectué sous 100-150°C et une pression de 0,1-0,6 MPa.

2. La méthode selon la revendication 1, **caractérisé par le fait que** l'ammoniac est ajouté en état gazeux et/ou liquide et/ou en forme d'une solution aqueuse.

3. La méthode de la séparation de gaz dissous à partir de la solution aqueuse de mélamine non purifiée par son soufflage à l'aide de la vapeur d'eau **caractérisé par le fait qu'**au cours du soufflage par la vapeur d'eau on ajoute en plus de l'ammoniac où l'ammoniac est ajouté en quantité de 0,01-1,0 kg / 1 kg de mélamine et où le soufflage par la vapeur d'eau est effectué sous 100-150°C et une pression de 0,1-0,6 MPa.

4. La méthode selon la revendication 3 **caractérisé par le fait que** l'ammoniac est ajouté en état gazeux et/ou liquide et/ou en forme d'une solution aqueuse.
